(19) Europäisches Patentamt / European Patent Office / Office européen des brevets

(11) **EP 3 605 369 B1**

(12) **EUROPEAN PATENT SPECIFICATION**

(45) Date of publication and mention
of the grant of the patent:
**28.08.2024  Bulletin 2024/35**

(21) Application number: **18788158.6**

(22) Date of filing: **20.04.2018**

(51) International Patent Classification (IPC):
**G16H 50/30** *(2018.01)*      **G16H 20/70** *(2018.01)*

(52) Cooperative Patent Classification (CPC):
**G16H 50/30; G16H 20/70**

(86) International application number:
**PCT/CN2018/083885**

(87) International publication number:
**WO 2018/192567 (25.10.2018 Gazette 2018/43)**

(54) **METHOD FOR DETERMINING EMOTIONAL THRESHOLD AND ARTIFICIAL INTELLIGENCE DEVICE**

VERFAHREN ZUR BESTIMMUNG EINER EMOTIONALEN SCHWELLE VORRICHTUNG FÜR KÜNSTLICHE INTELLIGENZ

PROCÉDÉ DE DÉTERMINATION DE SEUIL ÉMOTIONNEL ET DISPOSITIF D'INTELLIGENCE ARTIFICIELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.04.2017  CN 201710262505**

(43) Date of publication of application:
**05.02.2020  Bulletin 2020/06**

(73) Proprietor: **Huawei Technologies Co., Ltd.**
**Longgang District**
**Shenzhen, Guangdong 518129 (CN)**

(72) Inventors:
• **DONG, Mingjie**
  **Shenzhen, Guangdong 518129 (CN)**
• **HUANG, Kangmin**
  **Shenzhen, Guangdong 518129 (CN)**
• **SUN, Wenhua**
  **Shenzhen, Guangdong 518129 (CN)**

(74) Representative: **Maiwald GmbH**
**Elisenhof**
**Elisenstraße 3**
**80335 München (DE)**

(56) References cited:
**CN-A- 106 295 568     CN-A- 106 503 646**

**CN-A- 107 194 151     US-A1- 2015 235 655**

• **HWANG K S ET AL: "Reward shaping for reinforcement learning by emotion expressions", 2014 IEEE INTERNATIONAL CONFERENCE ON SYSTEMS, MAN, AND CYBERNETICS (SMC), IEEE, 5 October 2014 (2014-10-05), pages 1288 - 1293, XP032693785, DOI: 10.1109/SMC.2014.6974092**
• **WILFREDO JR. BADOY ET AL: "Q-Learning with Basic Emotions", ARXIV, 6 September 2016 (2016-09-06), pages 1 - 7, XP055664407, Retrieved from the Internet <URL:https://arxiv.org/ftp/arxiv/papers/1609/1609.01468.pdf> [retrieved on 20200203], DOI: arXiv:1609.01468v1**
• **XINYU HU ET AL: "A First Step Towards Behavioral Coaching for Managing Stress: A Case Study on Optimal Policy Estimation with Multi-stage Threshold Q-learning", AMIA ... ANNUAL SYMPOSIUM PROCEEDINGS. AMIA SYMPOSIUM, 1 January 2017 (2017-01-01), United States, pages 930 - 939, XP055664067, Retrieved from the Internet <URL:https://www.ncbi.nlm.nih.gov/pmc/articles/PMC5977571/pdf/2730191.pdf>**

Description

TECHNICAL FIELD

This application relates to the artificial intelligence field, and more specifically, to a method for determining a sentiment threshold and an artificial intelligence device.

BACKGROUND

With continuous development of artificial intelligence, a primary task for a new generation artificial intelligence system is possessing a sentiment connection capability for "sensibility", to meet people's common requirements in mentality and sentiment in a more humanized manner and gradually build trust and attachment.
In other words, artificial intelligence should not merely be an ordinary intelligence tool, and should not always develop along a rational way but should fall within an intersection area between sensibility and rationality.
Sentiment computing plays an important role in the development of artificial intelligence. A growing quantity of products with "sentiment" appear, but this is merely a start. Human emotion is an extremely complex issue, and artificial intelligence still has a long way to go.
The Article by HWANG K S ET AL, titled "Reward shaping for reinforcement learning by emotion expressions", 2014 IEEE INTERNATIONAL CONFERENCE ON SYSTEMS, MAN, AND CYBERNETICS (SMC), IEEE, (20141005), describes systems and methods non-expert learning system was proposed to guide the robots learn their behaviors by humans' emotional expressions.
The proposed system used interval fuzzy type-2 algorithm to recognize the human's facial expressions, which were captured by a web camera. Furthermore, emotion value (E-value), generated based on non-expert human's facial expressions, was applied to the reinforcement learning to train robots.
Currently, a user's sentiment is determined by the artificial intelligence device by comparing an obtained sensor parameter used to monitor physiological information of a user with a preset sentiment threshold. For example, if the obtained sensor parameter is greater than the sentiment threshold, it may be determined that a sentiment status of the user changes; or if the obtained sensor parameter is not greater than the sentiment threshold, it may be determined that a sentiment status of the user does not change.
In the foregoing technical solutions, the sentiment threshold is preset in the artificial intelligence device. Sentiment thresholds of different users may be different, and sentiment thresholds of a user in different scenarios may also be different. Therefore, how to determine sentiment thresholds to adapt to different users is a problem to be urgently resolved.

SUMMARY

This application provides a method for adapting a sentiment threshold to a specific user according to claim 1 and an artificial intelligence device, to optimize a sentiment threshold and improve communication efficiency and communication effects in different scenarios according to claim 6.
According to a first aspect, an embodiment of this application provides a method for determining a sentiment threshold and an artificial intelligence device. The method includes: step 1: determining, by an artificial intelligence device based on obtained monitoring information, that a sentiment status of a first user is a first sentiment state; step 2: obtaining, by the artificial intelligence device, N actions of a second user, where the second user is a user who communicates with the first user, and N is a positive integer greater than or equal to 1; step 3: determining, by the artificial intelligence device, a first action based on a Q-value table, where each Q value in the Q-value table corresponds to a sentiment state and an action, a Q value that corresponds to the first sentiment state and the first action is a largest value in N Q values in the Q-value table, and an $n^{th}$ Q value in the N Q values corresponds to the first sentiment state and an $n^{th}$ action in the N actions, where $n = 1, ..., N$; step 4: updating, by the artificial intelligence device, the Q value that corresponds to the first sentiment state and the first action in the Q-value table; and step 5: determining, by the artificial intelligence device, whether an updated Q value is greater than a preset threshold, and if the artificial intelligence device determines that the updated Q value is greater than the preset threshold, determining the sentiment threshold based on the monitoring information, or if the artificial intelligence device determines that the updated Q value is not greater than the preset threshold, repeating step 1 to step 5 until the sentiment threshold is determined, where that the updated Q value is greater than the preset threshold indicates that the sentiment status of the first user changes from the first sentiment state to a specific sentiment state. In the foregoing technical solution, the artificial intelligence device may optimize the sentiment threshold by using a Q-learning method, to improve communication efficiency and communication effects in different scenarios.
With reference to the first aspect, in a first possible implementation of the first aspect, the updating, by the artificial intelligence device, the Q value that corresponds to the first sentiment state and the first action in the Q-value table includes: updating, by the artificial intelligence device based on a first return rate, the Q value that corresponds to the first sentiment state and the first action in the Q-value table.

With reference to the first possible implementation of the first aspect, in a second possible implementation of the first aspect, the updating, by the artificial intelligence device based on a first return rate, the Q value that corresponds to the first sentiment state and the first action in the Q-value table includes: updating, by the artificial intelligence device by using the following formula, the Q value that corresponds to the first sentiment state and the first action in the Q-value table: $Q_{t+1}(s_{t+1},a_{t+1}) = (1-\lambda) Q_t(s_t,a_t) + \lambda[r_t + \gamma \max Q_t(s_t,a_t)]$, where $Q_{t+1}(s_{t+1},a_{t+1})$ represents the updated Q value that corresponds to the first sentiment state and the first action in the Q-value table, $\lambda$ represents learning strength, $Q_t(s_t,a_t)$ represents the Q value that is prior to the update and that corresponds to the first sentiment state and the first action in the Q-value table, $\gamma$ represents a discount factor, $r_t$ represents the first return rate, and $\max Q_t(s_t,a_t)$ represents a largest Q value that is prior to the update and that corresponds to the first sentiment state in the Q-value table.

With reference to any one of the first aspect, or the foregoing possible implementations of the first aspect, in a third possible implementation of the first aspect, the method further includes: determining, by the artificial intelligence device, a sentiment threshold level; and determining, by the artificial intelligence device, the preset threshold based on the sentiment threshold level. The optimized sentiment threshold can better meet a user's requirement by setting the sentiment threshold level, to improve prediction accuracy and improve communication efficiency and communication effects.

With reference to any one of the first aspect, or the foregoing possible implementations of the first aspect, in a fourth possible implementation of the first aspect, the method further includes: determining, by the artificial intelligence device, a sentiment threshold level; and the determining a sentiment threshold based on the monitoring information corresponding to the first sentiment state includes: determining the sentiment threshold based on the sentiment threshold level and the monitoring information. The optimized sentiment threshold can better meet a user's requirement by setting the sentiment threshold level, to improve prediction accuracy and improve communication efficiency and communication effects.

With reference to the third or the fourth possible implementation of the first aspect, in a fifth possible implementation of the first aspect, the determining, by the artificial intelligence device, a sentiment threshold level includes: determining, by the artificial intelligence device, the sentiment threshold level based on at least one of personalized factor information, conversation scenario information, external environment information, and input information of the first user. According to the foregoing technical solution, when determining the sentiment threshold level, the artificial intelligence device considers an objective condition (namely, at least one of the personalized factor information, the conversation scenario information, the external environment information, and the input information of the first user), to improve prediction accuracy.

With reference to any one of the first aspect, or the foregoing possible implementations of the first aspect, in a sixth possible implementation of the first aspect, the method further includes: determining, by the artificial intelligence device, whether current monitoring information is greater than the sentiment threshold when the artificial intelligence device determines the sentiment threshold; and sending indication information if it is determined that the current monitoring information is greater than the sentiment threshold, where the indication information is used to prompt that the sentiment status of the first user changes if the second user performs the first action. In this way, the artificial intelligence device can prompt in a timely manner based on the determined sentiment threshold, another user communicating with the user to avoid any action that can change the sentiment status of the user.

According to a second aspect, an embodiment of this application provides an artificial intelligence device. The artificial intelligence device includes units configured to perform any one of the first aspect or the possible implementations of the first aspect.

According to a third aspect, an embodiment of this application provides an artificial intelligence device. The artificial intelligence device includes a processor, a memory, and an input apparatus. The processor is configured to execute an instruction stored in the memory and perform, in combination with the memory and the input apparatus, steps in any one of the first aspect or the possible implementations of the first aspect.

Another aspect of this application provides a computer readable storage medium, where the computer readable storage medium stores an instruction, and when the instruction runs on a computer, the computer performs the methods according to the foregoing aspects.

Another aspect of this application provides a computer program product including an instruction, and when the computer program product runs on a computer, the computer performs the methods according to the foregoing aspects.

## BRIEF DESCRIPTION OF DRAWINGS

FIG. 1 is a schematic flowchart of a method for determining a sentiment threshold according to an embodiment of this application;
FIG. 2 is a structural block diagram of an artificial intelligence device according to an embodiment of this application; and
FIG. 3 is a structural block diagram of an artificial intelligence device according to an embodiment of this application.

## DESCRIPTION OF EMBODIMENTS

The following describes the technical solutions of this application with reference to the accompanying drawings.

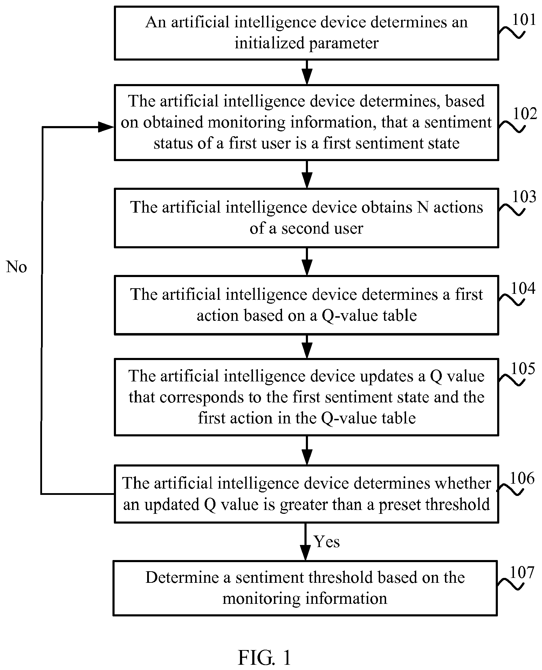

An artificial intelligence device in the embodiments of this application is any device that can implement a method shown in FIG. 1, and may be, for example, a computer or a robot.

FIG. 1 is a schematic flowchart of a method for determining a sentiment threshold according to an embodiment of this application.

101. An artificial intelligence device determines an initialized parameter, where the initialized parameter includes a Q-value table and a preset threshold.

Each Q value in the Q-value table corresponds to a sentiment state and an action. For example, Table 1 is an example of a Q-value table.

**Table 1**

|    | A1  | A2  | A3  | A4  | A5  |
|----|-----|-----|-----|-----|-----|
| S1 | Q11 | Q12 | Q13 | Q14 | Q15 |
| S2 | Q21 | Q22 | Q23 | Q24 | Q25 |
| S3 | Q31 | Q32 | Q33 | Q34 | Q35 |
| S4 | Q41 | Q42 | Q43 | Q44 | Q45 |

Table 1 is a Q-value table including four rows and five columns. The four rows respectively represent four sentiment states. The four sentiment states are respectively S1, S2, S3, and S4. The five columns respectively represent five actions. The five actions are respectively A1, A2, A3, A4, and A5. Each Q value in the Q-value table corresponds to an action and a sentiment state. For example, Q11 represents a Q value whose sentiment state is S1 and whose action is A1; Q12 represents a Q value whose sentiment state is S1 and whose action is A2; and so on.

Optionally, in an embodiment, all Q values in the Q-value table are zeros in an initial state.

102. The artificial intelligence device determines, based on obtained monitoring information, that a sentiment status of a first user is a first sentiment state.

Specifically, the artificial intelligence device may obtain the monitoring information. The monitoring information may be obtained by a monitoring device by monitoring data such as physiological data and action data of the first user. The monitoring information may be one or more pieces of information that can be used to determine a sentiment status of a user. For example, the monitoring information may include action data and/or physiological data. The action data may include one or more of speech, a facial expression, a gesture, a standing posture, and the like. The physiological data may include one or more of a heart rate, a pulse, skin conductance, and body temperature. It may be understood that if the monitoring information is data expressed non-digitally manner such as action data, the data may be expressed digitally for ease of processing.

The monitoring device is a device, an apparatus, or a sensor that can obtain the monitoring information, and the monitoring device may be one or more devices. For example, the monitoring device may include a speech recognition device, a facial expression recognition device, a heart rate sensor, a temperature sensor, and the like. For another example, the monitoring device may alternatively be one device, and the device has a speech recognition function, a facial expression recognition function, and functions of obtaining a heart rate and body temperature. The monitoring device may be integrated with the artificial intelligence device, or may be an independent device, which is not limited in this embodiment of this application.

The artificial intelligence device may directly determine the sentiment status of the first user based on the obtained monitoring information, or may determine the sentiment status of the first user after further processing the monitoring information, which is not limited in this embodiment of this application.

103. The artificial intelligence device determines N actions of a second user, where the second user is a user who communicates with the first user, and N is a positive integer greater than or equal to 1.

The action in this embodiment of this application is any action that is performed by the second user and that may be perceived by the first user, and includes but is not limited to speech data, a body movement, a facial expression action, and the like. For example, in a scenario of a dialog between the first user and the second user, the artificial intelligence device may obtain speech data of the second user. The speech data may include specific content, a tone, a speed, and other speech data of the second user. The artificial intelligence device may further obtain a body movement of the second user.

It may be understood that the second user may perform one or more actions in a dialog.

The artificial intelligence device may obtain the at least one action of the second user in a plurality of manners. For example, the artificial intelligence device may be provided with a built-in camera to obtain a body movement, a facial expression, and the like of the second user. The artificial intelligence device may also be provided with a built-in microphone to obtain the speech data of the second user. For another example, the camera and the microphone may alternatively be external devices, and the artificial intelligence device may obtain actions and speech data obtained by these external devices.

104. The artificial intelligence device determines a first action based on the Q-value table, where each Q value in the Q-value table corresponds to a sentiment state and an action, a Q value that corresponds to the first sentiment state and the first action is a largest value in N Q values in the Q-value table, and an $n^{th}$ Q value in the N Q values corresponds to the first sentiment state and an $n^{th}$ action in the N actions, where $n = 1, ..., N$.

Assuming that a first sentiment state that is of the first user and determined by the artificial intelligence device is S1, and the N actions that are of the second user and determined by the artificial intelligence device include A1, A2, and A5, where Q11 is less than Q12, and Q12 is less than Q15, the first action determined by the artificial intelligence device is A5.

105. The artificial intelligence device updates the Q value that corresponds to the first sentiment state and the first action in the Q-value table.

The updating the Q value that corresponds to the first sentiment state and the first action in the Q-value table includes: updating, based on a first return rate, the Q value that corresponds to the first sentiment state and the first action in the Q-value table.

The artificial intelligence device may determine the first return rate based on the first action and/or the first sentiment state.

Optionally, in some embodiments, the artificial intelligence device may determine the first return rate based on a preset return rate table. The return rate table may include a plurality of return rates. Each return rate in the return rate table corresponds to an action and a sentiment status. The return rate in the return rate table may be determined based on an empirical value.

Optionally, in some other embodiments, the artificial intelligence device may determine the first return rate according to a preset formula.

Optionally, in some embodiments, the artificial intelligence device may update, according to the following formula, the Q value that corresponds to the first sentiment state and the first action in the Q-value table:

$$Q_{t+1}\left(s_{t+1}, a_{t+1}\right) = \left(1 - \lambda\right)Q_t\left(s_t, a_t\right) + \lambda\left[r_t + \gamma \max Q_t\left(s_t, a_t\right)\right], \text{ (Formula 1.1)}$$

where $Q_{t+1}(s_{t+1}, a_{t+1})$ represents an updated Q value that corresponds to the first sentiment state and the first action in the Q-value table, $\lambda$ represents learning strength, $Q_t(s_t, a_t)$ represents the Q value that is prior to the update and that corresponds to the first sentiment state and the first action in the Q-value table, $\gamma$ represents a discount factor, $r_t$ represents the first return rate, and $\max Q_t(s_t, a_t)$ represents a largest Q value that is prior to the update and that corresponds to the first sentiment state in the Q-value table.

Optionally, in some other embodiments, the artificial intelligence device may update, according to the following formula, the Q value that corresponds to the first sentiment state and the first action in the Q-value table:

$$Q_{t+1}\left(s_{t+1}, a_{t+1}\right) = \gamma Q_t\left(s_t, a_t\right) + \lambda r_t, \text{ (Formula 1.2)}$$

where $Q_{t+1}(s_{t+1}, a_{t+1})$ represents the updated Q value that corresponds to the first sentiment state and the first action in the Q-value table, $\lambda$ represents learning strength, $Q_t(s_t, a_t)$ represents the Q value that is prior to the update and that corresponds to the first sentiment state and the first action in the Q-value table, $\gamma$ represents a discount factor, and $r_t$ represents the first return rate.

106. The artificial intelligence device determines whether an updated Q value is greater than a preset threshold. If the updated Q value is greater than the preset threshold, perform step 107, or if the updated Q value is not greater than the preset threshold, repeat step 102 to step 106 until a determined result in step 106 is that the updated Q value is greater than the preset threshold.

107. Determine a sentiment threshold based on the monitoring information.

Optionally, in some embodiments, the preset threshold is determined based on an empirical value.

Optionally, in some other embodiments, the preset threshold is determined based on a sentiment threshold level. The artificial intelligence device may first determine a sentiment threshold level, and then determine the preset threshold based on the sentiment threshold level.

Optionally, in some embodiments, the artificial intelligence device may determine the sentiment threshold level based on at least one of personalized factor information, conversation scenario information, external environment information, and input information of the first user. According to the foregoing technical solution, when determining the sentiment threshold level, the artificial intelligence device considers an objective condition (namely, at least one of the personalized factor information, the conversation scenario information, the external environment information, and the input information of the first user), to improve prediction accuracy.

The personalized factor information is used to indicate a user's personalized characteristic, for example, a user's personality. Specifically, different persons have particular differences in stimuli from external things and a retention time in each sentiment state, or spontaneous transition between different sentiment states. Optionally, the personalized factor information of the first user may be obtained by training personality information of the first user based on a basic personality template library. A personalized factor library may be trained based on an existing mature basic personality template library studied in the academic world, to learn and obtain personalized factors of different users. The personalized factor library can be used to simulate different persons' sentiment states to obtain change rules, to further improve the prediction accuracy.

For example, the artificial intelligence device may determine the sentiment threshold level based on the personalized factor information of the first user. For example, if the first user is insensitive to external things, the sentiment threshold level may be set to be relatively high, or if the first user is sensitive to external things, the sentiment threshold level may be set to be relatively low.

When the first user communicates with different persons, sentiment thresholds of the first user are different. For example, when communicating with a child, the first user is gentle and pleased, and a threshold triggering the sentiment status of the first user to change may be relatively high. For another example, when the first user communicates with some specific objects, the sentiment status of the first user fluctuates relatively greatly, and a threshold triggering the sentiment status of the first user to change may be relatively low. In this embodiment of this application, information related to the second user, for example, personalized factor information and identity information of the second user, is referred to as conversation scenario information.

Different external environments may also affect a sentiment status of a tested user. For example, a sentiment status change of the tested user varies between a terrified atmosphere and an environment of being at home. In this embodiment of this application, information that is used for a sentiment prediction module to construct an external environment scenario and unrelated to the second user is referred to as external environment information. The external environment information may include at least one item of the following information: weather information, geographical location information of the first user, and the like. For example, if the weather is good, the artificial intelligence device may set the sentiment threshold to be relatively high, or if the weather is bad, the artificial intelligence device may set the sentiment threshold to be relatively low.

In some other embodiments, the first user or the second user may directly enter a sentiment threshold level as required.

Optionally, in some embodiments, if the artificial intelligence device determines that related information (namely, personalized factor information, conversation scenario information, and external environment information) is never encountered new information, the sentiment threshold level may be set to be relatively high.

If the artificial intelligence device determines that the conversation scenario information is never encountered new conversation scenario information, the sentiment threshold level may be set to be relatively low.

A higher sentiment threshold level indicates a higher preset threshold determined based on the sentiment threshold level. Correspondingly, a lower sentiment threshold level indicates a lower preset threshold determined based on the sentiment threshold level. A curve of the change of the preset threshold corresponding to the sentiment threshold level may be linear or nonlinear, which is not limited in this embodiment of this application.

Optionally, in some embodiments, a correspondence between the sentiment threshold level and the preset threshold is predetermined. In some other embodiments, the preset threshold may be determined based on the sentiment threshold level and an empirical value.

Each Q value in the Q-value table may have a corresponding preset threshold, and preset thresholds of different Q values may be the same or different. It may be understood that the preset threshold in step 106 is a preset threshold of the Q value that corresponds to the first sentiment state and the first action.

Optionally, when the preset threshold is determined based on the sentiment threshold level, the artificial intelligence device may directly determine that the monitoring information is the sentiment threshold.

Optionally, in some embodiments, the preset threshold may be the empirical value. In this case, the artificial intelligence device may determine the sentiment threshold based on the sentiment threshold level and the monitoring information.

For example, the artificial intelligence device determines, after M periods, that an updated Q value is greater than the preset threshold, where M is a positive integer greater than or equal to 2. In this case, the artificial intelligence device may determine that monitoring information in an $(M-m)^{th}$ period is the sentiment threshold, where m is a positive integer greater than or equal to 1 and less than M. A value of m may be determined based on the sentiment threshold level. A higher sentiment threshold level indicates a larger value of m, and a lower sentiment threshold level indicates a smaller value of m.

For another example, the sentiment threshold is determined based on the monitoring information and the sentiment threshold level. For example, the monitoring information may be multiplied by a coefficient. A higher sentiment threshold level indicates a larger coefficient, and a lower sentiment threshold level indicates a smaller coefficient.

Certainly, in some embodiments, both the preset threshold and the sentiment threshold may be determined based on the sentiment threshold level. For a specific manner, refer to the foregoing embodiments. Details are not described herein.

In the technical solution shown in FIG. 1, the artificial intelligence device may optimize the sentiment threshold by using a Q-learning method, to improve communication efficiency and communication effects in different scenarios.

In addition, the optimized sentiment threshold can better meet a user's requirement by setting the sentiment threshold level, to improve prediction accuracy and improve communication efficiency and communication effects.

Further, when the artificial intelligence device determines the sentiment threshold, the method may further include: determining, by the artificial intelligence device, whether current monitoring information exceeds the sentiment threshold. If the current monitoring information exceeds the sentiment threshold, indication information may be sent. The indication information is used to prompt that the sentiment status of the first user changes if the second user performs the first action. In this way, the artificial intelligence device may prompt the second user in a timely manner with actions that may cause the sentiment status change of the first user. The second user may avoid corresponding actions that cause the sentiment status change of the first user. The artificial intelligence device may send the indication information in a plurality of manners, such as a voice prompt, a text prompt, and an image prompt, which is not limited in this embodiment of this application.

Further, the artificial intelligence device may further determine a difference between an updated Q value and a Q value that is prior to the update. If the difference is less than a preset difference, it may be determined that a Q value that corresponds to the first sentiment state and the first action in the Q-value table still uses the Q value that is prior to the update. If the difference is greater than the preset difference, it may be determined that a Q value that corresponds to the first sentiment state and the first action in the Q-value table is the updated Q value.

Optionally, in some embodiments, if the updated Q value exceeds a preset threshold of the Q value, it may indicate that the sentiment status of the user changes from a current sentiment status to a specific sentiment state. There is a preset threshold of a corresponding Q value and a sentiment threshold for a change from each sentiment state to another sentiment state. For example, when the updated Q value exceeds a first preset threshold of the Q value, it may indicate that the sentiment status of the user changes from happiness to astonishment. For another example, when the updated Q value exceeds a second preset threshold of the Q value, it may indicate that the sentiment status of the user changes from astonishment to anger. It may be understood that, a manner shown in FIG. 1 merely shows a method for determining a sentiment threshold. According to the method shown in FIG. 1, sentiment thresholds corresponding to different sentiment states may be determined.

In addition, the specific sentiment state may be an adjacent sentiment state of the current sentiment state, or may be a non-adjacent sentiment state. For example, a sentiment status changes from happiness to anger may experience the astonished state. The first preset threshold is less than the second preset threshold. In some embodiments, the preset threshold of the Q value may be the first preset threshold or the second preset threshold. In other words, in these embodiments, the intelligence device may set a sentiment threshold for each sentiment state of the first user. In some other embodiments, the preset threshold of the Q value may be directly set to the second preset threshold. In these embodiments, the intelligence device may only set a sentiment threshold for a concerned sentiment state (such as anger).

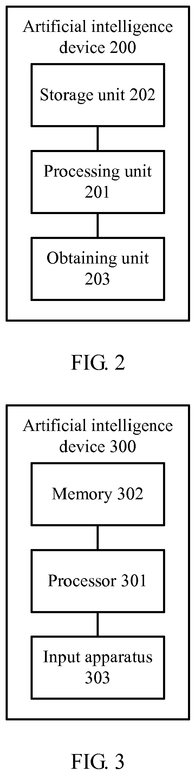

FIG. 2 is a structural block diagram of an artificial intelligence device according to an embodiment of this application. As shown in FIG. 2, an artificial intelligence device 200 may include: a processing unit 201, a storage unit 202, and an obtaining unit 203.

The obtaining unit 203 is configured to obtain N actions of a second user, where N is a positive integer greater than or equal to 1.

The storage unit 202 is configured to store a Q-value table.

The processor unit 201 is configured to perform the following steps:

step 1: determining, based on monitoring information, that a sentiment status of a first user is a first sentiment state;

step 2: obtaining the N actions obtained by the obtaining unit 203, where the second user is a user who communicates with the first user;

step 3: determining a first action based on the Q-value table stored in the storage unit 202, where each Q value in the Q-value table corresponds to a sentiment state and an action, a Q value that corresponds to the first sentiment state and the first action is a largest value in N Q values in the Q-value table, and an $n^{th}$ Q value in the N Q values corresponds to the first sentiment state and an $n^{th}$ action in the N actions, where n = 1, ..., N.

step 4: updating the Q value that corresponds to the first sentiment state and the first action in the Q-value table stored in the storage unit 202; and

step 5: determining whether the updated Q value is greater than a preset threshold, and if it is determined that the updated Q value is greater than the preset threshold, determining the sentiment threshold based on the monitoring information, or if it is determined that the updated Q value is not greater than the preset threshold, repeating step 1 to step 5 until the sentiment threshold is determined, where that the updated Q value is greater than the preset threshold indicates that the sentiment status of the first user changes from the first sentiment state to a specific sentiment state.

Optionally, in some embodiments, the processing unit 201 is specifically configured to update, based on a first return rate, the Q value that corresponds to the first sentiment state and the first action in the Q-value table stored in the storage unit 202.

Optionally, in some embodiments, the processing unit 201 is specifically configured to update, by using the following formula, the Q value that corresponds to the first sentiment state and the first action in the Q-value table stored in the storage unit 202:

$$ Q_{t+1}\left(s_{t+1}, a_{t+1}\right) = \left(1-\lambda\right) Q_t\left(s_t, a_t\right) + \lambda[r_t + \gamma \max Q_t\left(s_t, a_t\right)] $$

where $Q_{t+1}(s_{t+1},a_{t+1})$ represents the updated Q value that corresponds to the first sentiment state and the first action in the Q-value table, $\lambda$ represents learning strength, $Q_t(s_t,a_t)$ represents the Q value that is prior to the update and that corresponds to the first sentiment state and the first action in the Q-value table, $\gamma$ represents a discount factor, $r_t$ represents the first return rate, and max $Q_t(s_t,a_t)$ represents a largest Q value that is prior to the update and that corresponds to the first sentiment state in the Q-value table.

Optionally, in some embodiments, the processing unit 201 is further configured to determine a sentiment threshold level, and the processing unit 201 is further configured to determine the preset threshold based on the sentiment threshold level.

Optionally, in some embodiments, the processing unit 201 is further configured to determine a sentiment threshold level, and the processing unit 201 is specifically configured to determine the sentiment threshold based on the sentiment threshold level and the monitoring information.

Optionally, the processing unit 201 is specifically configured to determine the sentiment threshold level based on at least one of personalized factor information, conversation scenario information, external environment information, and input information of the first user.

Optionally, in some embodiments, the artificial intelligence device 200 may further include an output unit. The processing unit 201 may be further configured to: determine whether current monitoring information is greater than the sentiment threshold when the sentiment threshold is determined; and instruct the output unit to send out indication information if it is determined that the current monitoring information is greater than the sentiment threshold, where the indication information is used to prompt that the sentiment status of the first user changes if the second user performs the first action.

The storage unit 202 may be further configured to store the sentiment threshold level, the preset threshold, the sentiment threshold, and other information that are determined by the processing unit 201.

The processing unit 201 may be implemented by using a processor. The storage unit 202 may be implemented by using a memory. The obtaining unit 202 may be implemented by using an input device such as a microphone and a camera. The output unit may be implemented by using an output device such as a loudspeaker and a display.

The artificial intelligence device 200 shown in FIG. 2 can implement processes implemented in the method embodiment in FIG. 1. To avoid repetition, details are not described herein again.

FIG. 3 is a structural block diagram of an artificial intelligence device according to an embodiment of this application. As shown in FIG. 3, the artificial intelligence device 300 may include a processor 301, a memory 302, and an input apparatus 303. The memory 302 may be configured to store information such as a Q-value table, a sentiment threshold level, a preset threshold, and a sentiment threshold, and may be further configured to store a code, an instruction, and the like that are executed by the processor 301. Components in the robot 300 are coupled to each other by using a bus system. In addition to a data bus, the bus system further includes a power bus, a control bus, and a state signal bus.

The artificial intelligence device 300 shown in FIG. 3 can implement processes implemented in the method embodiment in FIG. 1. To avoid repetition, details are not described herein again.

A person of ordinary skill in the art may be aware that, in combination with the examples described in the embodiments disclosed in this specification, units and algorithm steps may be implemented by electronic hardware or a combination of computer software and electronic hardware. Whether the functions are performed by hardware or software depends on particular applications and design constraint conditions of the technical solutions. A person skilled in the art may use different methods to implement the described functions for each particular application, but it should not be considered that the implementation goes beyond the scope of this application.

It may be clearly understood by a person skilled in the art that, for the purpose of convenient and brief description, for a detailed working process of the foregoing system, apparatus, and unit, reference may be made to a corresponding process in the foregoing method embodiments, and details are not described herein again.

In the several embodiments provided in this application, it should be understood that the disclosed system, apparatus, and method may be implemented in other manners. For example, the described apparatus embodiment is merely an example. For example, the unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected according to actual requirements to achieve the objectives of the solutions of the embodiments.

In addition, functional units in the embodiments of this application may be integrated into one processing unit, or each of the units may exist alone physically, or two or more units are integrated into one unit.

All or some of the foregoing embodiments may be implemented by means of software, hardware, firmware, or any combination thereof. When software is used to implement the embodiments, the embodiments may be implemented completely or partially in a form of a computer program product. The computer program product includes one or more computer instructions. When the computer program instructions are loaded and executed on the computer, the procedure or functions according to the embodiments of this application are all or partially generated. The computer may be a general-purpose computer, a dedicated computer, a computer network, or other programmable apparatuses. The computer instructions may be stored in a computer-readable storage medium, or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (Digital Subscriber Line, DSL)) or wireless (for example, infrared, radio, and microwave, or the like) manner. The computer-readable storage medium may be any usable medium accessible by a computer, or a data storage device, such as a server or a data center, integrating one or more usable media. The usable medium may be a magnetic medium (for example, a soft disk, a hard disk, or a magnetic tape), an optical medium (for example, a DVD), a semiconductor medium (for example, a solid-state drive (Solid State Disk, SSD)), or the like.

The foregoing descriptions are merely specific implementations of this application, but are not intended to limit the protection scope, which is defined by the claims appended to this application.

**Claims**

1. A method for adapting a sentiment threshold to a specific user, wherein the method comprises:

   step 1: for a first user, determining (102), by an artificial intelligence device based on obtained action data and/or physiological data monitoring information, that a sentiment status of the first user is a first sentiment state;
   step 2: obtaining (103), by the artificial intelligence device, N actions of a second user, wherein the second user is a user who communicates with the first user, the actions are any actions that are performed by the second user and that may be perceived by the first user, and N is a positive integer greater than 1;
   step 3: determining (104), by the artificial intelligence device, a first action out of the N actions of the second user based on a Q-value table, wherein each Q value in the Q-value table corresponds to a sentiment state and an action, a Q value that corresponds to the first sentiment state and the first action is a largest value in N Q values in the Q-value table, and an $n^{th}$ Q value in the N Q values corresponds to the first sentiment state and an nth action in the N actions, wherein n = 1, ..., N;

step 4: updating (105), by the artificial intelligence device, the Q value that corresponds to the first sentiment state and the first action in the Q-value table; and

step 5: determining (106), by the artificial intelligence device, whether an updated Q value is greater than a preset threshold, and if the artificial intelligence device determines that the updated Q value is greater than the preset threshold, determining (107) a sentiment threshold based on the monitoring information, or if the artificial intelligence device determines that the updated Q value is not greater than the preset threshold, repeating step 1 to step 5 until the sentiment threshold is determined, wherein the updated Q value being greater than the preset threshold indicates that the sentiment status of the first user changes from the first sentiment state to a specific sentiment state when the second user performs the first action, wherein the updating, by the artificial intelligence device, the Q value that corresponds to the first sentiment state and the first action in the Q-value table comprises:

updating, by the artificial intelligence device based on a first return rate, the Q value that corresponds to the first sentiment state and the first action in the Q-value table, wherein the first return rate is the reward associated with the first sentiment state and the first action;

wherein the method further comprises: determining, by the artificial intelligence device, whether current monitoring information is greater than the sentiment threshold when the artificial intelligence device determines the sentiment threshold; and

sending indication information if it is determined that the current monitoring information is greater than the sentiment threshold, wherein the indication information is used to prompt that the sentiment status of the first user changes if the second user performs the first action.

2. The method according to claim 1, wherein the updating, by the artificial intelligence device based on a first return rate, the Q value that corresponds to the first sentiment state and the first action in the Q-value table comprises: updating, by the artificial intelligence device by using the following formula, the Q value that corresponds to the first sentiment state and the first action in the Q-value table:

$$Q_{t+1}\left(s_{t+1}, a_{t+1}\right) = \left(1-\lambda\right)Q_t\left(s_t, a_t\right) + \lambda[r_t + \gamma \max Q_t\left(s_t, a_t\right)]$$

wherein $Q_{t+1}$ ($s_{t+1}, a_{t+1}$) represents the updated Q value that corresponds to the first sentiment state and the first action in the Q-value table, $\lambda$ represents learning strength, $Q_t$ ($s_t, a_t$) represents the Q value that is prior to the update and that corresponds to the first sentiment state and the first action in the Q-value table, $\gamma$ represents a discount factor, $r_t$ represents the first return rate, and max $Q_t$ ($s_t, a_t$) represents a largest Q value that is prior to the update and that corresponds to the first sentiment state in the Q-value table.

3. The method according to any one of claims 1 to 2, wherein the method further comprises:

determining, by the artificial intelligence device, a sentiment threshold level; and
determining, by the artificial intelligence device, the preset threshold based on the sentiment threshold level.

4. The method according to any one of claims 1 to 2, wherein the method further comprises:

determining, by the artificial intelligence device, a sentiment threshold level; and
the determining a sentiment threshold based on the monitoring information comprises:
determining the sentiment threshold based on the sentiment threshold level and the monitoring information.

5. The method according to claim 3 or 4, wherein the determining, by the artificial intelligence device, a sentiment threshold level comprises:
determining, by the artificial intelligence device, the sentiment threshold level based on at least one of personalized factor information, conversation scenario information, external environment information, or input information of the first user.

6. An artificial intelligence device (200), wherein the artificial intelligence device comprises a processing unit (201), a storage unit (202), and an obtaining unit (203);

the obtaining unit is configured to obtain N actions of a second user, wherein N is a positive integer greater than 1;
the storage unit is configured to store a Q-value table; and
the processing unit is configured to perform the following steps:

step 1: for a first user, determining, based on obtained action data and/or physiological data monitoring information, that a sentiment status of the first user is a first sentiment state;
step 2: obtaining the N actions obtained by the obtaining unit, wherein the second user is a user who communicates with the first user and the actions are any actions that are performed by the second user and that may be perceived by the first user;
step 3: determining a first action out of the N actions of the second user based on the Q-value table stored in the storage unit, wherein each Q value in the Q-value table corresponds to a sentiment state and an action, a Q value that corresponds to the first sentiment state and the first action is a largest value in N Q values in the Q-value table, and an $n^{th}$ Q value in the N Q values corresponds to the first sentiment state and an $n^{th}$ action in the N actions, wherein n = 1, ..., N;
step 4: updating the Q value that corresponds to the first sentiment state and the first action in the Q-value table stored in the storage unit; and
step 5: determining whether an updated Q value is greater than a preset threshold, and if it is determined that the updated Q value is greater than the preset threshold, determining a sentiment threshold based on the monitoring information, or if it is determined that the updated Q value is not greater than the preset threshold, repeating step 1 to step 5 until the sentiment threshold is determined, wherein the updated Q value being greater than the preset threshold indicates that the sentiment status of the first user changes from the first sentiment state to a specific sentiment state when the second user performs the first action, wherein the processing unit is specifically configured to update, based on a first return rate, the Q value that corresponds to the first sentiment state and the first action in the Q-value table stored in the storage unit, wherein the first return rate is the reward associated with the first sentiment state and the first action;
wherein the artificial intelligence device further comprises an output unit;
the processing unit is further configured to: determine whether current monitoring information is greater than the sentiment threshold when the sentiment threshold is determined; and
if it is determined that the current monitoring information is greater than the sentiment threshold, instruct the output unit to send indication information, wherein the indication information is used to prompt that the sentiment status of the first user changes if the second user performs the first action.

7. The artificial intelligence device according to claim 6, wherein the processing unit is specifically configured to update, by using the following formula, the Q value that corresponds to the first sentiment state and the first action in the Q-value table stored in the storage unit:

$$Q_{t+1}\left(s_{t+1}, a_{t+1}\right) = \left(1 - \lambda\right) Q_t\left(s_t, a_t\right) + \lambda[r_t + \gamma \max Q_t\left(s_t, a_t\right)]$$

wherein $Q_{t+1}(s_{t+1}, a_{t+1})$ represents the updated Q value that corresponds to the first sentiment state and the first action in the Q-value table, $\lambda$ represents learning strength, $Q_t(s_t, a_t)$ represents the Q value that is prior to the update and that corresponds to the first sentiment state and the first action in the Q-value table, $\gamma$ represents a discount factor, $r_t$ represents the first return rate, and $\max Q_t(s_t, a_t)$ represents a largest Q value that is prior to the update and that corresponds to the first sentiment state in the Q-value table.

8. The artificial intelligence device according to any one of claims 6 to 7, wherein the processing unit is further configured to determine a sentiment threshold level; and
the processing unit is further configured to determine the preset threshold based on the sentiment threshold level.

9. The artificial intelligence device according to any one of claims 6 to 7, wherein the processing unit is further configured to determine a sentiment threshold level; and
the processing unit is specifically configured to determine the sentiment threshold based on the sentiment threshold level and the monitoring information.

10. The artificial intelligence device according to claim 8 or 9, wherein the processing unit is specifically configured to determine the sentiment threshold level based on at least one of personalized factor information, conversation scenario information, external environment information, and input information of the first user.

**Patentansprüche**

1. Verfahren zur Anpassung einer Stimmungsschwelle an einen bestimmten Benutzer,
   wobei das Verfahren Folgendes umfasst:

   Schritt 1: Bestimmen (102), für einen ersten Benutzer, durch eine Vorrichtung für künstliche Intelligenz basierend auf erlangten Überwachungsinformationen über Aktionsdaten und/oder physiologische Daten, dass ein Stimmungsstatus des ersten Benutzers ein erster Stimmungszustand ist;
   Schritt 2: Erlangen (103), durch die Vorrichtung für künstliche Intelligenz, von N Aktionen eines zweiten Benutzers, wobei der zweite Benutzer ein Benutzer ist, der mit dem ersten Benutzer kommuniziert, die Aktionen beliebige Aktionen sind, die durch den zweiten Benutzer durchgeführt werden und die durch den ersten Benutzer wahrgenommen werden können, und N eine positive ganze Zahl größer als 1 ist;
   Schritt 3: Bestimmen (104), durch die Vorrichtung für künstliche Intelligenz, einer ersten Aktion aus den N Aktionen des zweiten Benutzers basierend auf einer Q-Werte-Tabelle, wobei jeder Q-Wert in der Q-Werte-Tabelle einem Stimmungszustand und einer Aktion entspricht, ein Q-Wert, der dem ersten Stimmungszustand und der ersten Aktion entspricht, ein größter Wert in N Q-Werten in der Q-Werte-Tabelle ist und ein n-ter Q-Wert in den N Q-Werten dem ersten Stimmungszustand und einer $n^{ten}$ Aktion in den N Aktionen entspricht, wobei n = 1, ..., N;
   Schritt 4: Aktualisieren (105), durch die Vorrichtung für künstliche Intelligenz, des Q-Werts, der dem ersten Stimmungszustand und der ersten Aktion entspricht, in der Q-Werte-Tabelle; und
   Schritt 5: Bestimmen (106), durch die Vorrichtung für künstliche Intelligenz, ob ein aktualisierter Q-Wert größer als eine voreingestellte Schwelle ist, und, wenn die Vorrichtung für künstliche Intelligenz bestimmt, dass der aktualisierte Q-Wert größer als die voreingestellte Schwelle ist, Bestimmen (107) einer Stimmungsschwelle basierend auf den Überwachungsinformationen, oder, wenn die Vorrichtung für künstliche Intelligenz bestimmt, dass der aktualisierte Q-Wert nicht größer als die voreingestellte Schwelle ist, Wiederholen von Schritt 1 bis Schritt 5, bis die Stimmungsschwelle bestimmt ist, wobei der aktualisierte Q-Wert, der größer als die voreingestellte Schwelle ist, anzeigt, dass sich der Stimmungsstatus des ersten Benutzers von dem ersten Stimmungszustand zu einem bestimmten Stimmungszustand ändert, wenn der zweite Benutzer die erste Aktion durchführt, wobei das Aktualisieren, durch die Vorrichtung für künstliche Intelligenz, des Q-Werts, der dem ersten Stimmungszustand und der ersten Aktion entspricht, in der Q-Werte-Tabelle Folgendes umfasst:

   Aktualisieren, durch die Vorrichtung für künstliche Intelligenz basierend auf einer ersten Return-Rate, des Q-Werts, der dem ersten Stimmungszustand und der ersten Aktion entspricht, in der Q-Werte-Tabelle, wobei die erste Return-Rate die Belohnung ist, die dem ersten Stimmungszustand und der ersten Aktion zugeordnet ist;
   wobei das Verfahren ferner Folgendes umfasst: Bestimmen, durch die Vorrichtung für künstliche Intelligenz, ob aktuelle Überwachungsinformationen größer als die Stimmungsschwelle sind, wenn die Vorrichtung für künstliche Intelligenz die Stimmungsschwelle bestimmt; und
   Senden von Anzeigeinformationen, wenn bestimmt wird, dass die aktuellen Überwachungsinformationen größer als die Stimmungsschwelle sind, wobei die Anzeigeinformationen verwendet werden, um zu veranlassen, dass sich der Stimmungsstatus des ersten Benutzers ändert, wenn der zweite Benutzer die erste Aktion durchführt.

2. Verfahren nach Anspruch 1, wobei das Aktualisieren, durch die Vorrichtung für künstliche Intelligenz basierend auf einer ersten Return-Rate, des Q-Werts, der dem ersten Stimmungszustand und der ersten Aktion entspricht, in der Q-Werte-Tabelle Folgendes umfasst: Aktualisieren, durch die Vorrichtung für künstliche Intelligenz unter Verwendung der folgenden Formel, des Q-Werts, der dem ersten Stimmungszustand und der ersten Aktion entspricht, in der Q-Werte-Tabelle:

$$Q_{t+1}\ (s_{t+1},\ a_{t+1}) = (1 - \lambda)\ Q_t\ (s_t,\ a_t) + \lambda[r_t + \gamma \max Q_t\ (s_t,\ a_t)]$$

wobei $Q_{t+1}\ (s_{t+1}, a_{t+1})$ den aktualisierten Q-Wert, der dem ersten Stimmungszustand und der ersten Aktion entspricht, in der Q-Werte-Tabelle darstellt, $\lambda$ die Lernstärke darstellt, $Q_t\ (s_t, a_t)$ den Q-Wert, der vor der Aktualisierung liegt und der dem ersten Stimmungszustand und der ersten Aktion entspricht, in der Q-Werte-Tabelle darstellt, $\gamma$ einen Diskontierungsfaktor darstellt, $r_t$ die erste Return-Rate darstellt und $\max Q_t\ (s_t, a_t)$ einen größten Q-Wert, der vor der Aktualisierung liegt und der dem ersten Stimmungszustand entspricht, in der Q-Werte-Tabelle darstellt.

3. Verfahren nach einem der Ansprüche 1 bis 2, wobei das Verfahren ferner Folgendes umfasst:

Bestimmen, durch die Vorrichtung für künstliche Intelligenz, eines Stimmungsschwellenwerts; und
Bestimmen, durch die Vorrichtung für künstliche Intelligenz, der voreingestellten Schwelle basierend auf dem Stimmungsschwellenwert.

4. Verfahren nach einem der Ansprüche 1 bis 2, wobei das Verfahren ferner Folgendes umfasst:

Bestimmen, durch die Vorrichtung für künstliche Intelligenz, eines Stimmungsschwellenwerts; und
das Bestimmen einer Stimmungsschwelle basierend auf den Überwachungsinformationen Folgendes umfasst:
Bestimmen der Stimmungsschwelle basierend auf dem Stimmungsschwellenwert und den Überwachungsinformationen.

5. Verfahren nach Anspruch 3 oder 4, wobei das Bestimmen, durch die Vorrichtung für künstliche Intelligenz, einer Stimmungsschwellenwerts Folgendes umfasst:
Bestimmen, durch die Vorrichtung für künstliche Intelligenz, des Stimmungsschwellenwerts basierend auf mindestens einem von Informationen über personalisierte Faktoren, Informationen über ein Gesprächsszenario, Informationen über eine externe Umgebung oder Eingabeinformationen des ersten Benutzers.

6. Vorrichtung (200) für künstliche Intelligenz, wobei die Vorrichtung für künstliche Intelligenz eine Verarbeitungseinheit (201), eine Speichereinheit (202) und eine Erlangungseinheit (203) umfasst;

die Erlangungseinheit dazu konfiguriert ist, N Aktionen eines zweiten Benutzers zu erlangen, wobei N eine positive ganze Zahl größer als 1 ist;
die Speichereinheit dazu konfiguriert ist, eine Q-Werte-Tabelle zu speichern; und
die Verarbeitungseinheit dazu konfiguriert ist, die folgenden Schritte durchzuführen:

Schritt 1: für einen ersten Benutzer, Bestimmen, basierend auf erlangten Überwachungsinformationen über Aktionsdaten und/oder physiologische Daten, dass ein Stimmungsstatus des ersten Benutzers ein erster Stimmungszustand ist;
Schritt 2: Erlangen der N Aktionen, die durch Erlangungseinheit erlangt wurden, wobei der zweite Benutzer ein Benutzer ist, der mit dem ersten Benutzer kommuniziert, und die Aktionen beliebige Aktionen sind, die durch den zweiten Benutzer durchgeführt werden und durch den ersten Benutzer wahrgenommen werden können;
Schritt 3: Bestimmen einer ersten Aktion aus den N Aktionen des zweiten Benutzers basierend auf der Q-Werte-Tabelle, die in der Speichereinheit gespeichert ist, wobei jeder Q-Wert in der Q-Werte-Tabelle einem Stimmungszustand und einer Aktion entspricht, ein Q-Wert, der dem ersten Stimmungszustand und der ersten Aktion entspricht, ein größter Wert in N Q-Werten in der Q-Werte-Tabelle ist und ein $n$-ter Q-Wert in den N Q-Werten dem ersten Stimmungszustand und einer $n^{ten}$ Aktion in den N Aktionen entspricht, wobei $n = 1, ..., N$;
Schritt 4: Aktualisieren des Q-Werts, der dem ersten Stimmungszustand und der ersten Aktion entspricht, in der Q-Werte-Tabelle, die in der Speichereinheit gespeichert ist; und
Schritt 5: Bestimmen, ob ein aktualisierter Q-Wert größer als eine voreingestellte Schwelle ist, und wenn bestimmt wird, dass der aktualisierte Q-Wert größer als die voreingestellte Schwelle ist, Bestimmen einer Stimmungsschwelle basierend auf den Überwachungsinformationen, oder, wenn bestimmt wird, dass der aktualisierte Q-Wert nicht größer als die voreingestellte Schwelle ist, Wiederholen von Schritt 1 bis Schritt 5, bis die Stimmungsschwelle bestimmt ist, wobei der aktualisierte Q-Wert, der größer als die voreingestellte Schwelle ist, anzeigt, dass sich der Stimmungsstatus des ersten Benutzers von dem ersten Stimmungszustand zu einem bestimmten Stimmungszustand ändert, wenn der zweite Benutzer die erste Aktion durchführt,
wobei die Verarbeitungseinheit speziell dazu konfiguriert ist, basierend auf einer ersten Return-Rate, den Q-Wert, der dem ersten Stimmungszustand und der ersten Aktion entspricht, in der Q-Werte-Tabelle zu aktualisieren, die in der Speichereinheit gespeichert ist, wobei die erste Return-Rate die Belohnung ist, die dem ersten Stimmungszustand und der ersten Aktion zugeordnet ist;
wobei die Vorrichtung für künstliche Intelligenz ferner eine Ausgabeeinheit umfasst;
die Verarbeitungseinheit ferner zu Folgendem konfiguriert ist:

Bestimmen, ob aktuelle Überwachungsinformationen größer als die Stimmungsschwelle sind, wenn die Stimmungsschwelle bestimmt wird; und
wenn bestimmt wird, dass die aktuellen Überwachungsinformationen größer als die Stimmungsschwelle sind, Anweisen der Ausgabeeinheit, Anzeigeinformationen zu senden, wobei die Anzeigeinformationen verwendet werden, um zu veranlassen, dass sich der Stimmungsstatus des ersten Benutzers ändert, wenn der zweite Benutzer die erste Aktion durchführt.

7. Vorrichtung für künstliche Intelligenz nach Anspruch 6, wobei die Verarbeitungseinheit speziell dazu konfiguriert ist, unter Verwendung der folgenden Formel, den Q-Wert, der dem ersten Stimmungszustand und der ersten Aktion entspricht, in der Q-Werte-Tabelle zu aktualisieren, die in der Speichereinheit gespeichert ist:

$$Q_{t+1}\ (s_{t+1},\ a_{t+1})\ =\ (1\ -\ \lambda)\ Q_t\ (s_t,\ a_t)\ +\ \lambda[r_t\ +\ \gamma\ \max\ Q_t\ (s_t,\ a_t)]$$

wobei $Q_{t+1}$ ($s_{t+1}$, $a_{t+1}$) den aktualisierten Q-Wert, der dem ersten Stimmungszustand und der ersten Aktion entspricht, in der Q-Werte-Tabelle darstellt, $\lambda$ die Lernstärke darstellt, $Q_t$ ($s_t$, $a_t$) den Q-Wert, der vor der Aktualisierung liegt und der dem ersten Stimmungszustand und der ersten Aktion entspricht, in der Q-Werte-Tabelle darstellt, $\gamma$ einen Diskontierungsfaktor darstellt, $r_t$ die erste Return-Rate darstellt und max $Q_t$ ($s_t$, $a_t$) einen größten Q-Wert, der vor der Aktualisierung liegt und der dem ersten Stimmungszustand entspricht, in der Q-Werte-Tabelle darstellt.

8. Vorrichtung für künstliche Intelligenz nach einem der Ansprüche 6 bis 7, wobei die Verarbeitungseinheit ferner dazu konfiguriert ist, einen Stimmungsschwellenwert zu bestimmen; und
die Verarbeitungseinheit ferner dazu konfiguriert ist, die voreingestellte Schwelle basierend auf dem Stimmungsschwellenwert zu bestimmen.

9. Vorrichtung für künstliche Intelligenz nach einem der Ansprüche 6 bis 7, wobei die Verarbeitungseinheit ferner dazu konfiguriert ist, einen Stimmungsschwellenwert zu bestimmen; und
die Verarbeitungseinheit speziell dazu konfiguriert ist, die Stimmungsschwelle basierend auf dem Stimmungsschwellenwert und den Überwachungsinformationen zu bestimmen.

10. Vorrichtung für künstliche Intelligenz nach Anspruch 8 oder 9, wobei die Verarbeitungseinheit speziell dazu konfiguriert ist, den Stimmungsschwellenwert basierend auf mindestens einem von Informationen über personalisierte Faktoren, Informationen über ein Gesprächsszenario, Informationen über eine externe Umgebung und Eingabeinformationen des ersten Benutzers zu bestimmen.

## Revendications

1. Procédé pour adapter un seuil de sentiment à un utilisateur spécifique,
dans lequel le procédé comprend :

étape 1 : pour un premier utilisateur, la détermination (102), par un dispositif d'intelligence artificielle sur la base de données d'action obtenues et/ou d'informations de surveillance de données physiologiques, qu'un état de sentiment du premier utilisateur est un premier état de sentiment ;
étape 2 : l'obtention (103), par le dispositif d'intelligence artificielle, de N actions d'un second utilisateur, dans lequel le second utilisateur est un utilisateur qui communique avec le premier utilisateur, les actions sont toutes actions qui sont exécutées par le second utilisateur et qui peuvent être perçues par le premier utilisateur, et N est un nombre entier positif supérieur à 1 ;
étape 3 : la détermination (104), par le dispositif d'intelligence artificielle, d'une première action parmi les N actions du second utilisateur sur la base d'une table de valeurs Q, dans lequel chaque valeur Q dans la table de valeurs Q correspond à un état de sentiment et une action, une valeur Q qui correspond au premier état de sentiment et la première action est la plus grande valeur parmi les valeurs N Q dans la table de valeurs Q, et une $n^{ième}$ valeur Q dans les valeurs N Q correspond au premier état de sentiment et une $n^{ième}$ action parmi les N actions, dans lequel n = 1, ..., N ;
étape 4 : la mise à jour (105), par le dispositif d'intelligence artificielle, de la valeur Q qui correspond au premier état de sentiment et à la première action dans la table de valeurs Q ; et
étape 5 : le fait de déterminer (106), par le dispositif d'intelligence artificielle, si une valeur Q mise à jour est supérieure à un seuil prédéfini, et si le dispositif d'intelligence artificielle détermine que la valeur Q mise à jour est supérieure au seuil prédéfini, la détermination (107) d'un seuil de sentiment sur la base des informations de surveillance, ou si le dispositif d'intelligence artificielle détermine que la valeur Q mise à jour n'est pas supérieure au seuil prédéfini, la répétition de l'étape 1 à l'étape 5 jusqu'à ce que le seuil de sentiment soit déterminé, dans lequel la valeur Q mise à jour étant supérieure au seuil de sentiment indique que l'état de sentiment du premier utilisateur passe du premier état de sentiment à un état de sentiment spécifique lorsque le second utilisateur exécute la première action,
dans lequel la mise à jour, par le dispositif d'intelligence artificielle, de la valeur Q qui correspond au premier état de sentiment et à la première action dans la table de valeurs Q comprend :

> la mise à jour, par le dispositif d'intelligence artificielle sur la base d'un premier taux de retour, de la valeur Q qui correspond au premier état de sentiment et à la première action dans la table de valeurs Q, dans lequel le premier taux de retour est la récompense associée au premier état de sentiment et à la première action ;
> dans lequel le procédé comprend en outre : le fait de déterminer, par le dispositif d'intelligence artificielle, si les informations de surveillance actuelles sont supérieures au seuil de sentiment lorsque le dispositif d'intelligence artificielle détermine le seuil de sentiment ; et
> l'envoi d'informations d'indication s'il est déterminé que les informations de surveillance actuelles sont supérieures au seuil de sentiment, dans lequel les informations d'indication sont utilisées pour inciter à ce que l'état de sentiment du premier utilisateur change si le second utilisateur exécute la première action.

**2.** Procédé selon la revendication 1, dans lequel la mise à jour, par le dispositif d'intelligence artificielle sur la base d'un premier taux de retour, de la valeur Q qui correspond au premier état de sentiment et à la première action dans la table de valeurs Q comprend : la mise à jour, par le dispositif d'intelligence artificielle à l'aide de la formule suivante, de la valeur Q qui correspond au premier état de sentiment et à la première action dans la table de valeurs Q :

$$Q_{t+1}(s_{t+1}, a_{t+1}) = (1 - \lambda) Q_t(s_t, a_t) + \lambda[r_t + \gamma \max Q_t(s_t, a_t)]$$

dans lequel $Q_{t+1}(s_{t+1}, a_{t+1})$ représente la valeur Q mise à jour qui correspond au premier état de sentiment et à la première action dans la table de valeurs Q, $\lambda$ représente la force d'apprentissage, $Q_t(s_t, a_t)$ représente la valeur Q antérieure à la mise à jour et qui correspond au premier état de sentiment et à la première action dans la table de valeurs Q, $\gamma$ représente un facteur d'actualisation, $r_t$ représente le premier taux de retour et $\max Q_t(s_t, a_t)$ représente une valeur Q la plus élevée antérieure à la mise à jour et qui correspond au premier état de sentiment dans la table de valeurs Q.

**3.** Procédé selon l'une quelconque des revendications 1 à 2, dans lequel le procédé comprend en outre :

> la détermination, par le dispositif d'intelligence artificielle, d'un niveau de seuil de sentiment ; et
> la détermination, par le dispositif d'intelligence artificielle, du seuil prédéfini sur la base du niveau de seuil de sentiment.

**4.** Procédé selon l'une quelconque des revendications 1 à 2, dans lequel le procédé comprend en outre :

> la détermination, par le dispositif d'intelligence artificielle, d'un niveau de seuil de sentiment ; et
> la détermination d'un seuil de sentiment sur la base des informations de surveillance comprend :
> la détermination du seuil de sentiment sur la base du niveau de seuil de sentiment et des informations de surveillance.

**5.** Procédé selon la revendication 3 ou 4, dans lequel la détermination, par le dispositif d'intelligence artificielle, d'un niveau de seuil de sentiment comprend :
la détermination, par le dispositif d'intelligence artificielle, du niveau de seuil de sentiment sur la base d'au moins l'une parmi des informations de facteur personnalisées, des informations de scénario de conversation, des informations d'environnement externe ou des informations d'entrée du premier utilisateur.

**6.** Dispositif d'intelligence artificielle (200), dans lequel le dispositif d'intelligence artificielle comprend une unité de traitement (201), une unité de stockage (202) et une unité d'obtention (203) ;

> l'unité d'obtention est configurée pour obtenir N actions d'un second utilisateur, dans lequel N est un nombre entier positif supérieur à 1 ;

l'unité de stockage est configurée pour stocker une table de valeurs Q ; et
l'unité de traitement est configurée pour exécuter les étapes suivantes :

étape 1 : pour un premier utilisateur, la détermination, sur la base de données d'action obtenues et/ou d'informations de surveillance de données physiologiques, qu'un état de sentiment du premier utilisateur est un premier état de sentiment ;

étape 2 : l'obtention des N actions obtenues par l'unité d'obtention, dans lequel le second utilisateur est un utilisateur qui communique avec le premier utilisateur et les actions sont toutes actions qui sont exécutées par le second utilisateur et qui peuvent être perçues par le premier utilisateur ;

étape 3 : la détermination d'une première action parmi les N actions du second utilisateur sur la base d'une table de valeurs Q stockée dans l'unité de stockage, dans lequel chaque valeur Q dans la table de valeurs Q correspond à un état de sentiment et une action, une valeur Q qui correspond au premier état de sentiment et la première action est la plus grande valeur parmi les valeurs N Q dans la table de valeurs Q, et une $n^{\text{ième}}$ valeur Q dans les valeurs N Q correspond au premier état de sentiment et une $n^{\text{ième}}$ action parmi les N actions, dans lequel n = 1, ..., N ;

étape 4 : la mise à jour de la valeur Q qui correspond au premier état de sentiment et à la première action dans la table de valeurs Q stockée dans l'unité de stockage ; et

étape 5 : le fait de déterminer si une valeur Q mise à jour est supérieure à un seuil prédéfini, et s'il est déterminé que la valeur Q mise à jour est supérieure au seuil prédéfini, la détermination d'un seuil de sentiment sur la base des informations de surveillance, ou s'il est déterminé que la valeur Q mise à jour n'est pas supérieure au seuil prédéfini, la répétition de l'étape 1 à l'étape 5 jusqu'à ce que le seuil de sentiment soit déterminé, dans lequel la valeur Q mise à jour étant supérieure au seuil de sentiment indique que l'état de sentiment du premier utilisateur passe du premier état de sentiment à un état de sentiment spécifique lorsque le second utilisateur exécute la première action,

dans lequel l'unité de traitement est spécifiquement configurée pour mettre à jour, sur la base d'un premier taux de retour, la valeur Q qui correspond au premier état de sentiment et à la première action dans la table de valeurs Q stockée dans l'unité de stockage, dans lequel le premier taux de retour est la récompense associée au premier état de sentiment et à la première action ;

dans lequel le dispositif d'intelligence artificielle comprend en outre une unité de sortie ;

l'unité de traitement est en outre configurée pour : déterminer si des informations de surveillance actuelles sont supérieures au seuil de sentiment lorsque le seuil de sentiment est déterminé ; et

s'il est déterminé que les informations de surveillance actuelles sont supérieures au seuil de sentiment, ordonner à l'unité de sortie d'envoyer des informations d'indication, dans lequel les informations d'indication sont utilisées pour inciter à ce que l'état de sentiment du premier utilisateur change si le second utilisateur exécute la première action.

7. Dispositif d'intelligence artificielle selon la revendication 6, dans lequel l'unité de traitement est spécifiquement configurée pour mettre à jour, à l'aide de la formule suivante, la valeur Q qui correspond au premier état de sentiment et à la première action dans la table de valeurs Q stockée dans l'unité de stockage :

$$Q_{t+1}\,(s_{t+1},\ a_{t+1}) = (1 - \lambda)\,Q_t\,(s_t,\ a_t) + \lambda[r_t + \gamma\,\max Q_t\,(s_t,\ a_t)]$$

dans lequel $Q_{t+1}\,(s_{t+1}, a_{t+1})$ représente la valeur Q mise à jour qui correspond au premier état de sentiment et à la première action dans la table de valeurs Q, $\lambda$ représente la force d'apprentissage, $Q_t\,(s_t, a_t)$ représente la valeur Q antérieure à la mise à jour et qui correspond au premier état de sentiment et à la première action dans la table de valeurs Q, $\gamma$ représente un facteur d'actualisation, $r_t$ représente le premier taux de retour et $\max Q_t\,(s_t, a_t)$ représente une valeur Q la plus élevée antérieure à la mise à jour et qui correspond au premier état de sentiment dans la table de valeurs Q.

8. Dispositif d'intelligence artificielle selon l'une quelconque des revendications 6 à 7, dans lequel l'unité de traitement est en outre configurée pour déterminer un niveau de seuil de sentiment ; et
l'unité de traitement est en outre configurée pour déterminer le seuil prédéfini sur la base du niveau de seuil de sentiment.

9. Dispositif d'intelligence artificielle selon l'une quelconque des revendications 6 à 7, dans lequel l'unité de traitement est en outre configurée pour déterminer un niveau de seuil de sentiment ; et
l'unité de traitement est spécifiquement configurée pour déterminer le seuil de sentiment sur la base du niveau de seuil de sentiment et des informations de surveillance.

10. Dispositif d'intelligence artificielle selon la revendication 8 ou 9, dans lequel l'unité de traitement est spécifiquement configurée pour déterminer le niveau de seuil de sentiment sur la base d'au moins l'une parmi les informations de facteur personnalisées, des informations de scénario de conversation, des informations d'environnement externe et des informations d'entrée du premier utilisateur.

An artificial intelligence device determines an initialized parameter ⟩101

The artificial intelligence device determines, based on obtained monitoring information, that a sentiment status of a first user is a first sentiment state ⟩102

The artificial intelligence device obtains N actions of a second user ⟩103

The artificial intelligence device determines a first action based on a Q-value table ⟩104

The artificial intelligence device updates a Q value that corresponds to the first sentiment state and the first action in the Q-value table ⟩105

No

The artificial intelligence device determines whether an updated Q value is greater than a preset threshold ⟩106

Yes

Determine a sentiment threshold based on the monitoring information ⟩107

FIG. 1

Artificial intelligence device 200

Storage unit 202

Processing unit 201

Obtaining unit 203

FIG. 2

Artificial intelligence device 300

Memory 302

Processor 301

Input apparatus 303

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Reward shaping for reinforcement learning by emotion expressions. **HWANG K S et al.** 2014 IEEE INTERNATIONAL CONFERENCE ON SYSTEMS, MAN, AND CYBERNETICS (SMC). IEEE, 05 October 2014